(19) Europäisches Patentamt
European Patent Office
Office européen des brevets

(11) **EP 1 334 717 B1**

(12) **EUROPEAN PATENT SPECIFICATION**

(45) Date of publication and mention
of the grant of the patent:
**30.07.2008 Bulletin 2008/31**

(51) Int Cl.:
*A61K 9/48* (2006.01)    *A61K 47/14* (2006.01)
*A61K 9/127* (2006.01)

(21) Application number: **03008447.9**

(22) Date of filing: **17.08.2000**

(54) **Pharmaceutical Compositions For Oral And Topical Administration**

Pharmazeutische Zusammensetzungen zur oralen und topischen Verabreichung

Compositions pharmaceutiques pour administration orale et topique

(84) Designated Contracting States:
**AT BE CH CY DE DK ES FI FR GB GR IE IT LI LU MC NL PT SE**
Designated Extension States:
**AL LT LV MK RO SI**

(30) Priority: **17.08.1999 GB 9919288**

(43) Date of publication of application:
**13.08.2003 Bulletin 2003/33**

(62) Document number(s) of the earlier application(s) in accordance with Art. 76 EPC:
**00951765.7 / 1 210 119**

(73) Proprietor: **IVAX Pharmaceuticals s.r.o.**
**747 70 Opava 9 (CZ)**

(72) Inventors:
• **Andrysek, Tomas**
**747 05 Opava 5 (CZ)**
• **Stuchlik, Milan**
**747 05 Opava 5 (CZ)**
• **Vrana, Ales**
**747 05 Opava 5 (CZ)**
• **Jegorov, Alexander**
**373 16 Dobra Voda (CZ)**
• **Stuchlik, Josef**
**747 63 Hrabyni (CZ)**
• **Matha, Vladimir**
**370 00 Ceske Budejovice (CZ)**

(74) Representative: **King, Lawrence**
**D. Young & Co**
**120 Holborn**
**London EC1N 2DY (GB)**

(56) References cited:
**WO-A-98/10747**    **WO-A-98/40051**
**WO-A-99/00002**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

This invention relates to pharmaceutical formulations including, as the active ingredient, substances which are poorly soluble in water, for example therapeutically active cyclosporins, taxoides and taxanes.

Cyclosporins are a group of monocyclic, poly-N-methylated undecapeptides, which are naturally produced as secondary metabolites by certain fibrous fungi; especially of general Tolypocladium and Cylindrocarpon. Some therapeutically useful cyclosporin can be prepared by partial synthesis or by special fermentation procedures.

Ciclosoporin (Cyclosporin A) is the first natural substance having selective immunosuppressive effect on lymphoid cells, especially T lymphocytes. It also influences functions of other cells of the immune system to a great extent.

Systemically administered cyclosporin is used therapeutically in organ transplantations or transplantations of bone-marrow. Cyclosporin can be employed for treating a wide variety of autoimmune diseases with inflammatory etiology and also as anti-parasitic agents.

Certain cyclosporins without immunosuppressive activity exhibit an inhibitor effect towards replication of the HIV-1 virus and can be employed in therapy for treatment and prevention of AIDS or AIDS related complex. The group of cyclosporins also include chemomodulators useful for influencing cross resistance of tumour cells to cytostatics.

Bioavailability of cyclosporin is influenced, on one hand, by specific properties of this group of substances, but also by the composition and properties of the particular dosage form. An important role in formulating therapeutic compositions containing cyclosporin is played by their high lipophilicity.

Solubility of these active substances in water typically does not exceed 25 $\mu$g/ml, which value is approximately 100 times lower than needed for regular absorption in the organism. The marked lipophilicity of cyclosporin is evidenced by the values of their partition coefficients P in the system n-octanol/water. For cyclosporin, values of log P = 2.08 to 2.99 have been reported.

To achieve acceptable bioavailability of cyclosporins formulations which are used in practice form dispersion systems and are characterised by the presence of a hydrophilic phase, a hydrophobic phase and a tensoactive component. The resulting dispersions are either classic emulsions or optically transparent microemulsions. Commercially available compositions for oral administration are known under the trade names Sandimunn®, Sandimunn®-Neoral, Consupren®, Implanta®, Imusporin® as described in GB-A-2015339, GB-A-2222770, GB-A-2270842 and GB-A-2278780.

Modifications of the preceding systems, where the hydrophilic base is omitted and replaced by partial esters of fatty acids with polyols like propylene glycol, glycerol or sorbitol, are described in GB-A-2228198.

DE-A-4322826 discloses, as the carrier system for drugs poorly soluble in water, a composition containing polyglyceryl esters of fatty acids as a co-tenside to non-ionic tensides having HLB higher than 10, in the presence of a triacyl glycerol as the lipophilic component.

Formulations containing cyclosporins in a vehicle comprising propylene glycol, mixed mono-, di- and triglyceride and a hydrophilic tenside, disclosed in GB-A-2248615, are typical microemulsion preconcentrates of the oil-in-water type.

According to biopharmaceutical classification, cyclosporins belong to class IV, ie substances whose solubility in water is bad and bioavailability is poor (G L Amidon, Biopharmaceutics Drug Classification and International Drug Regulation, Capsgel Library, Bornem 1996, p 15 - 30).

Taxoides are a group of natural substances isolated from some strains of Taxus. Taxoides demonstrate antineoplastic effects by influencing cellular mitosis. They are diterpenic substances containing taxanic cyclic grouping with a 4-membered oxitanic ring and an esteric side chain in position $C_{13}$. Natural paclitaxel and its semisynthetic derivative docetaxel are used for treatment of tumours. Taxanes are even less soluble in water than cyclosporins. Immediately after preparation, paclitaxel solubility in water ranges about 5 $\mu$g/ml, however, paclitaxel hydrates which are formed on standing have an equilibrium concentration which is lower by an order of magnitude (0.3 - 0.6 $\mu$g/ml).

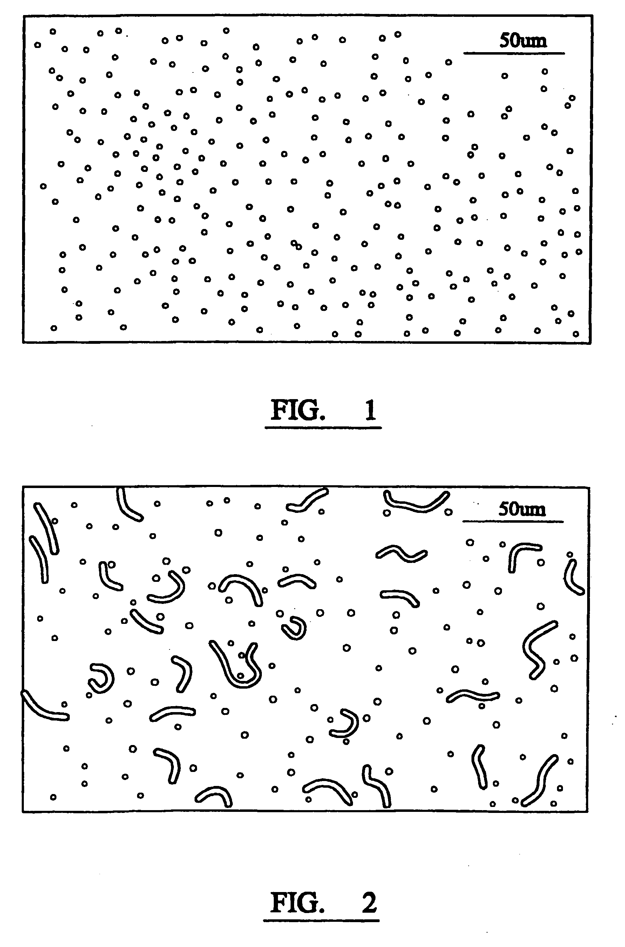

Compositions based on polyglycerol acylesters are known from the patent literature, eg WO98/05309. Pharmaceutical compositions for internal application containing cyclosporin as active ingredient and a carrier consisting of one or more partial esters of fatty acids with di- to decaglycerol and partial pentaglycerol to pentadecaglycerol acylesters are disclosed. Compositions prepared this way enable a skilled person to make a dispersion of emulsion type with an average particle size about 1 - 2 $\mu$m after dilution. The particles are of spherical character as shown in Figure 1. However, achievement of high bioavailability remains a problem.

Similarly, WO97/26003 discloses use of polyglycerol acylesters. Besides the above mentioned polyglycerolesters, the vehicle contains glycerol monoacylesters and optional substances selected from anhydrohexosdimethyl derivatives and/or polyethylene glycerols. The formulation can also contain other substances which improve the stability of the vehicle and lipoamino acids which are suitable especially for topical products. These compositions provide slightly dispersing systems containing spherical particles.

Other systems utilising polyglycerol esters with fatty acids are microemulsions. In EP-A-670715 or EP-A-334777, esters of fatty acids with polyglycerols are used for pharmaceutical or cosmetic microemulsions or compositions forming microemulsions. As defined in eg Lachman et al; Theory and Practice of Industrial Pharmacy, Lea & Febiger, Philadelphia 1970, p 463, a microemulsion is a clear dispersion of oil-in-water or water-in-oil having a size of dispersed particles in the range 100 - 600 Å. Dispersed particles in a microemulsion are composed of nanodrops or micellar aggregates of the dispersed phase in the dispersion medium. The shape of dispersed particles is mostly spherical.

Similarly, CZ-A-283516 describes use of polyglycerol acylesters as one of the components of vehicle which forms lyotropic liquid crystals in contact with an aqueous phase. In accordance with this specification and other patents (eg EP-A-314689 or EP-A-126751), only pharmaceutical compositions based on systems providing lyotropic liquid crystals are suitable and advantageous for formulations of biologically active substances which dissolve in the given system and/or have hydrophobic character. At the same time the capability of formation of a liquid crystal phase in vivo after application into the gastrointestinal tract is associated with high bioavailability of hydrophobic pharmaceutical compositions.

According to a draft of the article Cyclosporine Modified Capsules for USP 23, published in Pharmaceopeial Forum Volume 24, Number 3, 1998, p 6155, high bioavailability of cyclosporin is caused by dispersion of a pharmaceutical composition in the form of a pre-concentrate after administration of a microemulsion into GI tract. The draft recommends to test whether the dispersion arising after dilution of such composition provides particles of mean size 50 nm in the dispersed phase. This topic is discussed in several patents which however do not disclose use of polyglycerol esters of higher fatty acids.

A first aspect of the present invention relates to a pharmaceutical formulation for oral or topical administration including

a) 0.1 to 30.0% of one or more hydrophobic active ingredients;
b) 0.1 to 60.0% of one or more gelators selected from polyglycerol esters of fatty acids of formula (1)

$$CH_2OR\text{-}CHOR\text{-}CH_2O\text{-}[CH_2CHOR\text{-}CH_2O\text{-}]_NCH_2\text{-}CHOR\text{-}CH_2OR \qquad (1)$$

wherein n is an integer from 4 to 13 and R is H or CO.R' wherein R' is $C_{8\text{-}22}$ saturated, unsaturated or hydroxylated alkyl and wherein at least one group R is not hydrogen; having a HLB value not less than 10;
c) 0.1 to 60.0% of one or more gel-creating substances selected from polyglycerol esters of fatty acids and/or unsaturated fatty acids of formula (2)

$$CH_2OR\text{-}CHOR\text{-}CH_2O\text{-}[CH_2CHOR\text{-}CH_2O]_NCH_2\text{-}CHOR\text{-}CH_2OR \qquad (2)$$

wherein n is an integer from 0-10 and R = H or CO.R" wherein R" is $C_{8\text{-}22}$ saturated, unsaturated or hydroxylated alkyl, and wherein while at least one group R is not hydrogen; having a HLB value not greater than 9;
d) 1.0 to 60% of one or more co-gelator substances selected from triglyceride macrogol glycerol esters, partial glycerides or fatty acids or macrogol esters of fatty acids in which the average quantity of reacted ethylene oxide in the synthesis of these substances ranges between 50 to 150 mols and concurrently the ratio between components b) and d) is from 0.1:1 to 10:1;
e) 5.0 to 30% of one or more $C_2$ to $C_4$ alcohols;

wherein the above percentages are selected to total 100%;
and wherein upon dilution with water the formulation forms a dispersion of polymorphous gel particles having a dimension of 0.2 to 500 $\mu$m.

In a preferred embodiment, the ratio of a:c and/or a:e is in the range 0.001:1 to 10:1.

In another preferred embodiment, R' is $C_{16\text{-}18}$ saturated or unsaturated alkyl.

In yet another preferred embodiment, R is selected from the group consisting of oleates, linoleate stearate, linolate, myristate, laurate and mixtures thereof.

In another preferred embodiment, component b) is selected from: polyglyceryl-10-esters of fatty acids.

In yet another preferred embodiment, R" is $C_{16\text{-}18}$ saturated or unsaturated alkyl.

In another preferred embodiment, R is selected from the group of oleate, linoleate, stearate, isostearate, linolate, myristate, laurate and mixtures thereof.

In yet another preferred embodiment, component c) is selected from: polyglyceryl-3-esters of oleic acid.

In another preferred embodiment, component d) is macrogol glycol hydrogenated castor oil.

In yet another preferred embodiment, component b) is selected from: polyglyceryl-10-esters of oleic acid; component c) is selected from polyglyceryl-3-esters of oleic acid; and component d) is macrogol (1760) glycerol hydrogenated caster oil.

In another preferred embodiment, component a) is selected from cyclosporins especially cyclosporin A, cyclosporin D or cyclosporin G, wherein the ratio of components a:c+e is 1.001:1 to 1.5:1.

In yet another preferred embodiment, the alcohol is ethanol.

In another preferred embodiment, the pharmaceutical formulation further includes excipients to modify the physical, chemical, microbial stability, organoleptic or physical processing properties of the formulation.

A second aspect of the present invention relates to a pharmaceutical dosage form comprising a gelatin capsule containing a formulation as hereinbefore described.

In preferred formulations a minimum number of excipients are used. This results in economy of manufacture and regulatory requirements. A single compound from each of groups b) to e) is preferred.

The invention also provides use of a formulation in accordance with the second aspect of this invention for preparation of a dosage form for administration of a class IV substance.

It has been surprisingly found out that high bioavailability of cyclosporins and taxanes after oral application can be achieved using a system neither based on liquid crystals nor a microemulsion. It was also found that a system prepared in accordance with the present invention does not result in a dispersion of the emulsion type. Unexpectedly it has been found that particles which are formed spontaneously or almost spontaneously on mixing of the phases have a non-spherical character. At the same time, no sign of anisotropic grouping of molecules was found even if the particles formed exhibited a dramatic increase in viscosity. From these findings it appears that it is a dispersion in water of particles having gel-like properties.

In this specification particles of gel-like character are to be understood as those whose stable shape or conformation in the dispersion is non-spherical. Non-spherical particles are those having at least two different perpendicular dimensions.

In this specification a gel emulsion (GEM) is to be understood as a dispersion of particles of gel character in an aqueous phase.

A pre-concentrate of gel emulsion (PRO-GEM) is to be understood as a composition which results in a gel emulsion after dilution or in contact with an aqueous phase.

The formation of gel particles is caused by interaction between a hydrophilic gelator (an agent which causes formation of gel) and a lipophilic gel-creating phase. Such a composition may contain components which participate in the formation of a particulate gel structure and which facilitate spontaneous dispersion in an aqueous medium. It may also contain components which ensure oxidative or microbial stability, mask the taste, adjust the appearance or facilitate dissolution of active ingredients in the mixture. The composition may also contain components which adjust viscosity.

Pharmaceutical compositions in accordance with the present invention may be used to formulate active substances from class IV according to the biopharmaceutical classification. Also advantages are obtained when substances from class II and III are used.

Percentages and amounts used in this specification are by weight unless indicated otherwise.

In preferred formulations the ratio of a : c and/or a : e is in the range 0.001 : 1 to 10 : 1.

In contrast particles in liquid-liquid emulsions are generally spherical in shape. Particles of the present invention may have a substantial proportion, for example more than half with a non-spherical shape, for example an ellipsoid, rod-like or string-like shape. Preferably more than half of the particles by weight are elongate having a length more than twice their width or diameter. Formulations of this invention may have a particle size distribution with a median dimension in the range 1 to 100 $\mu$m, preferably 5 to 20 $\mu$m. Formulations may contain individual particles with a dimension up to 10 $\mu$m or more, for example 20 to 50 $\mu$m.

The formulations of the present invention may be made by mixing for example my manual stirring or shaking in vitro. Liquid formulations may be mixed with water, milk or other drink before administration. Higher speed stirring is less convenient but may be used, particularly to give smaller particle sizes, for example about 200 nm if desired.

Dosage forms comprising a gel-emulsion preconcentrate, eg in capsules, are mixed with aqueous phase in the GI tract. Sufficient shear forces are applied in the GI tract to form the polymorphous particles of the present invention.

Pharmaceutical compositions in accordance with the present invention may be characterised in that after dilution by mixing with an aqueous phase in ratio from approx 1 : 5 (composition : aqueous phase) to approx 1 : 100, a dispersion of gel particles in water with mean size of particles between 0.2 - 500 $\mu$m is obtained. Such dispersion may be referred to as a gel emulsion (GEM).

Gel emulsion pre-concentrates (PRO-GEM) may be administered in the form of a pre-concentrate or in single-dose dosage forms such as capsules.

Component a) includes biologically active ingredients which are insufficiently soluble in water for conventional formulation and so their bioavailability is low. According to this biopharmaceutical classification, these are substances of group 2 and 4, with low water solubility. These substances include immunosuppressives, antitumour chemotherapeutical agents, substances influencing saccharide metabolism, peptides and lipids, agents influencing the calcium channel, non-steroidal antiflogistics and vitamins.

Immunosuppressives are hydrophobic compounds and include N-methylated cyclic undecapeptides. Cyclosporins are preferably used, especially ciclosporin (also known as Ciclosporin or Cyclosporin A), [Nva][2] - ciclosporin (cyclosporin G) and [Melle][4] - ciclosporin. Non-immunosuppressive cyclosporines can also be used, eg [3'ketoMB-mt][1]-[Val][2]-ciclosporin. Various pharmacopoeias have referred to these compounds using different spellings. In this specification these compounds and derivatives thereof are conveniently referred to by the name cyclosporin. Other immunosuppressives can be used too, eg macrolides produced by grampositive Streptomyces bacteria (rapamycine, tacrolimus) or their derivatives.

Antitumour chemotherapeutic agents include taxanes, preferably docetaxel or paclitaxel.

Other biologically active ingredients which may be formulated in accordance with this invention may be selected from: diclofenac, ibuprofen, nifedipine, triamcinolone, tocopherol etc. In accordance with the present invention, the compositions can contain as much as 30% of the active ingredient.

Component b) which may be considered as a gelator is selected from polyglycerol esters of fatty acids, of general formula (I)

$$CH_2OR\text{-}CHOR\text{-}CH_2O\text{-}[CH_2CHOR\text{-}CH_2O\text{-}]_NCH_2\text{-}CHOR\text{-}CH_2OR \qquad (1)$$

where n is an integer from 4 - 13 and R = H or CO.R$^1$ wherein R$^1$ is C$_{8\text{-}22}$ saturated, unsaturated or hydroxylated alkyl and wherein at least one group R is not hydrogen.

Preferred components b) are polyglycerol esters and partial esters of medium or long chain fatty acids. These preferably have a HLB value not less than 10.

Polyglycerol esters with fatty acids are generally prepared by either partial or full esterification of polyglycerols by corresponding fatty acids or trans-esterification of vegetable oils with polyglycerol. Each polyglycerol monoester may be characterised by a saponification number. The level of polymerization is best indicated by the hydroxyl number. Polyglycerol esters with HLB value greater than about 10 may be considered to be hydrophilic. Polyglycerol esters with a HLB value less than about 9 may be considered lipophilic. Substances suitable for the components b) include the following:

| Name (INCI) | Number of glycerol units | HLB |
|---|---|---|
| Polyglycerol-6-monolaurate | 6 | 14.5 |
| Polyglyceryl-10-monolaurate | 10 | 15.5 |
| Polyglyceryl-10-monomyristate | 10 | 14.0 |
| Polyglyceryl-10-monostearate | 10 | 12.0 |
| Polyglyceryl-10-mono-dioleate | 10 | 11.0 |
| Polyglyceryl-10-diisostearate | 10 | 10.0 |
| Polyglyceryl-6-monomyristate | 6 | 11.0 |
| Polyglyceryl-8-monooleate | 8 | 11.0 |
| Polyglyceryl-10-monooleate | 10 | 12.0 |

The above mentioned polyglycerols esters are available from Nikko Chemicals Co under the trade name NIKKOL®, Durkee Foods under the trade name SANTONE® and from Th. Goldschmidt under the trade mark ISOLAN® or Abitec Corp under the trade name CAPROL®. Commercially available polyglyceryl esters may be mixtures containing predominantly the named ester or a mixture of esters having equivalent properties as determined for example by the hydroxyl value.

Polyglycerols esters of components b) and c) for use in the compositions of this invention preferably meet the following purity requirements:

acid no = max 6; heavy metals content = max 10 ppm; water content = max 2%; content of Na salts of fatty acids = max 2% (as Na stearate);

total ash = max 1%.

Preferred gelator compounds b) are selected from polyglyceryl esters of C$_{12\text{-}22}$ saturated, unsaturated or hydoxylated fatty acids including myristate, laurate, oleates, stearate, linoleate and linolate. C$_{16\text{-}22}$ acids are especially preferred. Most preferably C$_{16\text{-}18}$, that is stearate, oleates, laurate, linoleate and linolate. Mixtures may be used. Oleate esters or mixtures thereof are most preferred.

Triglyceryl esters of these acids, in which N = 1, have been found to be particularly suitable, especially for formulation of cyclosporins.

Component c), which may be considered as a gel-creating substance, is selected from polyglycerol esters of fatty acids and/or unsaturated fatty alcohols, and is preferably of general formula (2)

$$CH_2OR\text{-}CHOR\text{-}CH_2O\text{-}[CH_2CHOR\text{-}CH_2O]_NCH_2\text{-}CHOR\text{-}CH_2OR \qquad (2)$$

wherein n is an integer from 0 - 10 and R = H or CO.R'' wherein R'' is $C_{8-22}$ saturated, unsaturated or hydroxylated alkyl, and wherein while at least one group R is not hydrogen.

Preferred components c) are polyglycerol esters and partial esters of fatty acids and/or fatty alcohols. Preferred components c) have a HLB value not greater than 9. Substances suitable for components c) include the following:

| Name (INCI) | Number of glycerol units | HLB |
|---|---|---|
| Polyglyceryl-3-monooleate | 3 | 6.5 |
| Polyglyceryl-6-dioleate | 6 | 8.5 |
| Polyglyceryl-10-tetraoleate | 10 | 6.2 |
| Polyglyceryl-10-decaoleate | 10 | 3.5 |
| Polyglyceryl-2-monostearate | 2 | 5.0 |
| Polyglyceryl-10-pentastearate | 10 | 3.5 |

The above mentioned polyglycerols esters are available from Nikko Chemicals Co under the name NIKKOL®; or Abitec Corp under the trade name CAPROL®.

Preferred components c) include gel-creating substances selected from polyglycerol esters of fatty acids and/or unsaturated fatty alcohols is in accordance with the present invention a substance especially selected from $C_{8-22}$ unsaturated fatty alcohols. Preferably oleyl alcohol (9-octadecen-1 ol) can be used for example meeting the following purity requirements:

$$Mr = 268,49; \text{ refractive index} = 1,458 - 1,460; \text{ acid no} < 1; \text{ hydroxyl no} = 205 - 215; \text{ iodine no} = 85 - 95.$$

Preferred gel-creating components c) are selected from polyglyceryl esters of $C_{8-22}$ saturated, unsaturated or hydroxylated fatty acids, including myristate, laurate, oleates, stearate, linoleate and linolate. $C_{8-18}$ acids are preferred, $C_{8-16}$ acids being more preferred, including laurate, oleates and myristate. Mixtures may be employed. Oleate is the most preferred.

Polyglyceryl-10-esters of these acids, in which N = 8, have been found to be particularly suitable, especially for formulation of cyclosporins.

Component d), which may be considered to be a co-gelator, may be selected from: macrogolglycerol esters of fatty acids. These include esters of $C_{8-22}$ saturated or unsaturated fatty acids with macrogol glycerols.

Especially preferred are macrogol glycerols with vegetable oils eg ricine oil, both hydrogenated and unhydrogenated, almond or maize oil. They are generally prepared by reaction of various quantities of ethylene oxide and the appropriate type of oil under known conditions. Especially preferred are the following substances characterised by the number of reacted ethylene oxide mols (1 + m + n + x + y + z) and HLB value.

| | (1+m+n+x+y+z) | HLB |
|---|---|---|
| macrogol(1540) ricine-oleic glyceride | 35 | 12-14 |
| macrogol(1760) hydrogenated ricine-oleic glyceride | 40 | 12.5-16 |
| macrogol(2200) hydrogenated ricine-oleic glyceride | 50 | 13.5 |
| macrogol(2640) hydrogenated ricine-oleic glyceride | 60 | 14.5 |
| macrogol(3520) hydrogenated ricine-oleic glyceride | 80 | 15 |
| macrogol(4400) hydrogenated ricine-oleic glyceride | 100 | 16.5 |
| macrogol(2640 almond-oleic glyceride | 60 | 15 |
| macrogol(2640) maize-oleic glyceride | 60 | 15 |

Characteristic physical and chemical parameters of the above mentioned substances are:

acid no ≤ 2; hydroxyl no = 40 - 60; iodine no < 1*; saponification no =

40 - 70; water content ≤ 3%;

(*- for macrogol(1540) ricine-oleic glyceride = 28 - 32).

These substances are commercially available under the trade names eg Cremophor®, Nikkol®, Simulsol®, Mapeg®, Crovol®.

Special mixed mono- and d- macrogolesters of mono-, di- and triacylglycerol commercially available under the trade name Gelucire® are also preferred. Especially preferred products are available under the name Gelucire® 50/13 and 44/14. Preferred physicochemical properties are:

acid no < 2,00; saponification no = 65 - 95; iodine no < 2; hydroxyl no =

36 - 56; peroxide no < 6; alkaline impurities < 80 ppm; free glycerol <

3,00 %.

Alternative compositions preferred for use as compound d) are macrogolesters of fatty acids eg macrogol(660)-12-hydroxystearate commercially available under the trade name Solutol® HS 15 having an acid no < 1; water content < 0.5%; saponification no = 53 - 63 and hydroxyl no = 90 - 110.

Component d) is usually present in the compositions in an amount of 1 - 60 %, preferably in the range 5 - 50 % and most preferably 15 - 50% and most preferably 15 - 40 %.

Component e) is selected from $C_2$- $C_4$ alkanols, preferably ethyl alcohol of pharmaceopoeial quality. Alternative alkanols include isomers of propenol and buterol. Mixtures may be employed. In topical applications, propan-2-ol, or 2-methyl-1-propanol, are preferred.

Other excipients which can be employed in compositions of the present invention are those which influence physicochemical and microbial stability (eg antioxidants, antimicrobial additives such as tocopherol, methyl paraben), organoleptic properties (eg taste correctors based on natural or nature identical aromas) or physical properties which may limit processing (eg viscosity or melting point). The following can be included among such substances: water or other pharmaceutically acceptable solvents, hydrophilic colloids eg selected from derivatives of cellulose, chitosans, alginate, polycarbophile etc.

Compositions based on a gel pre-concentrate may be characterised in that they disperse into particles of gel character primarily of irregular shape after application into an aqueous medium. High bioavailability of such compositions is associated with bioadhesion. As a result of their amphilicity, these particles are less liable to coalescence and may be homogenously dispersed in an aqueous medium. In contact with a lipophilic surface they remain on the surface and so provide a sufficient concentration gradient to enable drug penetration through the membrane due to their viscosity and adhesivity.

The invention is further described by means of example but not in any limitative sense with reference to the accompanying drawings of which:

Figure 1 is a photomicrograph of a dispersion in accordance with WO98/05309;
Figure 2 is a photomicrograph of a dispersion in accordance with the present invention;
Figure 3 is a graph showing blood levels of cyclosporin in Example 6; and

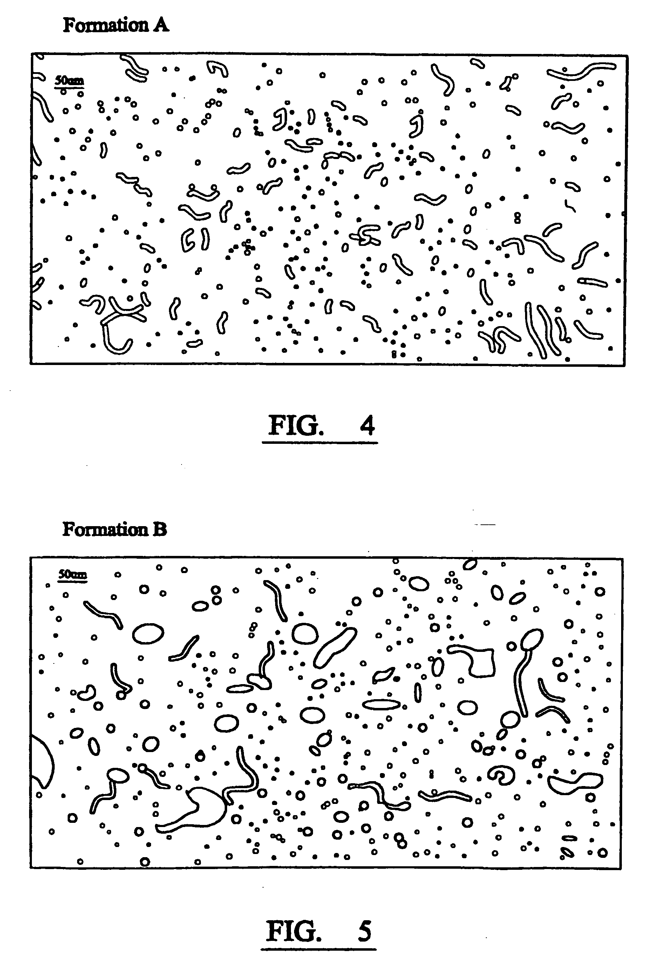

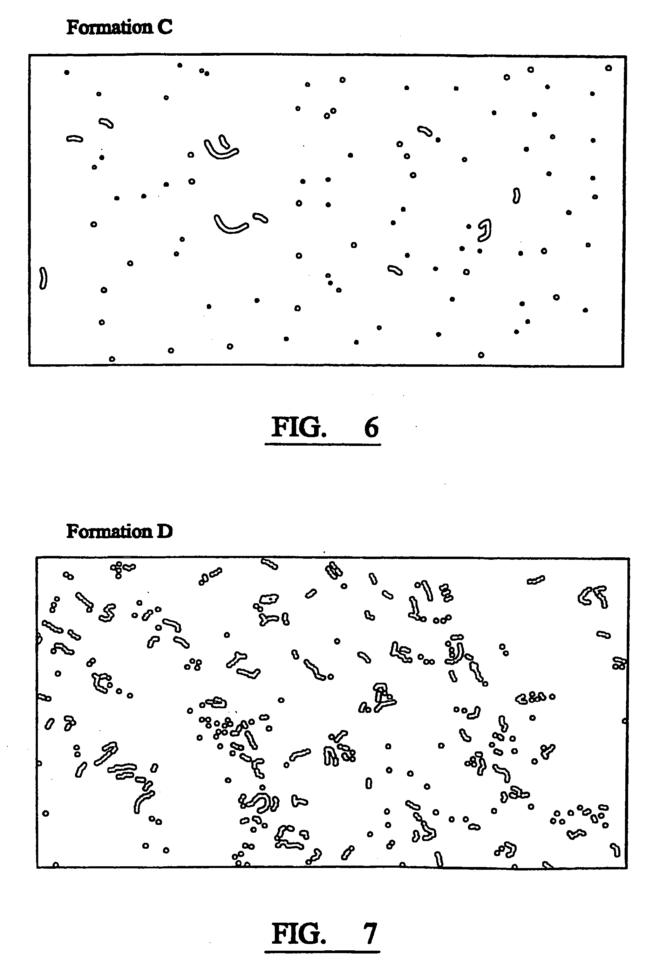

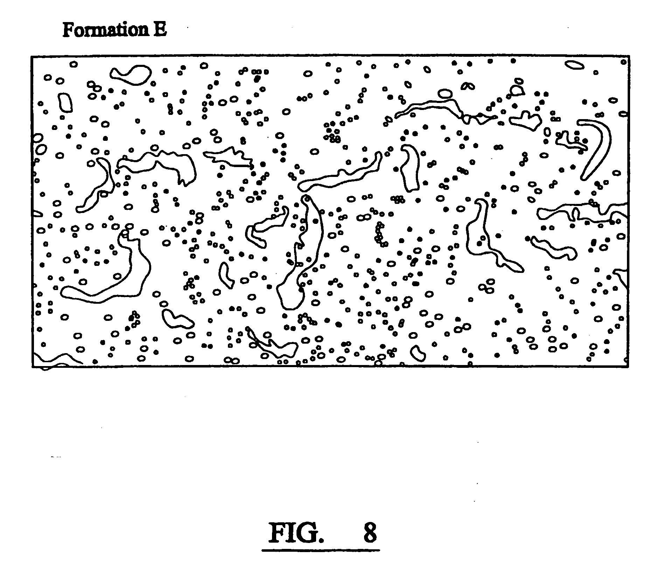

Figures 4 to 8 are photomicrographs of further dispersions in accordance with this invention.

Example 1

Cyclosporine-Containing Solution for Oral or Topical Application:

The following ingredients were employed.

| | | | |
|---|---|---|---|
| a) | cyclosporin A | 3600 g |
| b) | polyglycerol-10-mono-dioleate | 7200 g |
| c) | oleyl alcohol | 7200 g |

(continued)

| | | | |
|---|---|---|---|
| d) | macrogol(1760) hydrogenated ricine-oleic glyceride | 14400 g |
| e) | ethanol | 4000 g |
| f) | D-$\alpha$-tocopherol | 180 g |

Composition a) was mixed with compositions e) and c). The whole mixture was then homogenized until the active ingredient was dissolved. Then, compositions b) and d) and any other auxiliary ingredients were added. After complete homogenization the resulting solution was filtered through a hydrophobic membrane GVHP (Millipore) of porosity 0.2 - 5.0 $\mu$m into a gasproof vessel under an inert atmosphere. When required for use the filtered solution was packed under an inert atmosphere into 50 ml bottles equipped with gas-proof stoppers.

Example 2

Hard Gelatin Capsules of Size "Elongated 0"

The following ingredients were employed.

| | | |
|---|---|---|
| a) | cyclosporin A | 50.0 mg |
| b) | polyglyceryl-10-monooleate | 100.0 mg |
| c) | polyglyceryl-3-monooleate | 15.0 mg |
| d) | macrogol(2640) hydrogenated ricine-oleic glyceride | 140.0 mg |
| e) | ethanol | 80.0 mg |

The fill for hard gelatin capsules was prepared using working procedure identical to that of Example 1 and filled into hard gelatin capsules of size "EO".

Example 3

Cyclosporine Containing Solution for Oral Application

The following ingredients were employed.

| | | |
|---|---|---|
| a) | cyclosporin | 5.00 g |
| b) | polyglyerol(10) oleate | 9.50 g |
| c) | polyglyceryl(3) oleate | 15.50 g |
| d) | POE(40) hydrogenated castor oil (macrogol(1760) hydrogenated ricine-oleic glyceride) | 14.00 g |
| e) | absolute ethanol | 6.00 g |

Components were mixed and homogenised until the active ingredient was dissolved, followed by filtration and packaging in 50 ml bottles as described in Example 1, to provide an oral solution with 100 mg/ml dosage.

Example 4

Soft Gelatin Capsules

The following ingredients were employed.

Composition of Fill:

| | | |
|---|---|---|
| a) | cyclosporin | 100,00 mg |
| b) | polyglycerol(10) oleates | 210,00 mg |
| c) | polyglycerol(3) oleates | 350,00 mg |
| d) | POE(40) hydrogenated castor oil | 315.00 mg |
| e) | ethanol | 135,00 mg |

The fill for soft gelatin capsules was prepared by a procedure similar to that of Example 1. The gelatin capsules were prepared by mixing purified water, glycerol, sorbitol and gelatin. Homogenisation of the solution, addition of the colouring agents and production of 100 mg dosage capsules in conventional manner.

Example 5

Soft Gelatin Capsules of Size Oblong 20:

The following ingredients were employed.

| | | | |
|---|---|---|---|
| a) | cyclosporin A | 100.0 mg |
| b) | polyglyceryl-6-monolaurate | 120.0 mg |
| c) | polyglyceryl-10-tetraoleate | 410.0 mg |
| d) | Gelucire 50/13 | 300.0 mg |
| e) | ethanol | 170.0 mg |

The fill for soft gelatin capsules was prepared by a procedure identical to that of Example 1. The fill was filtered into a 20 1 stainless-steel vessel equipped with a gas-proof stopper. The fill was kept in inert atmosphere between filtration and encapsulation. Encapsulation was carried out using a conventional procedure into standard type of gelatin mixture.

Example 6

Hard HPMC Capsules (Shionogi Oualicaps) of Size 3:

The following ingredients were employed.

| | | | |
|---|---|---|---|
| a) | cyclosporin A | 25.0 mg |
| b) | polyglyceryl-10-myristate | 50.0 mg |
| c) | polyglyceryl-10-pentastearate | 70.0 mg |
| d) | macrogol(2640) almond-oleic glyceride | 75.0 mg |
| e) | ethanol | 30.0 mg |

Composition a) was mixed with compositions e) and b). The mixture was heated to 40 - 50°C and homogenised until composition a) was dissolved. Then, composition d) was added. Finally, composition c) was added. The mixture was continuously mixed. The temperature of the mixture did not exceed 60°C during preparation. After complete dissolution and homogenization of all ingredients the product is filtered through a pre-filter and filled into hard cellulose capsules (eg supplied by Syntapharm) of size 3.

Example 7

Visualisation of Gel Emulsion

Pre-concentrates in accordance with patent application WO98/05309 Example 1 and as disclosed in Example 1 of this invention were each diluted with water in ratio 1 : 20 (product: water) and dispersed on a laboratory shaker (IKA HS - B20) for 10 minutes at temperature 25 ± 1°C. Pictures of the dispersed samples were taken by means of a COHU camera connected to an optical microscope. The pictures were evaluated by means of software LUCIA™ (Laboratory Imaging Inc). Photomicrography of a dispersion of the emulsion type in accordance with WO98/05309 is shown in Figure 1. Photomicrography of a dispersion of the type of gel emulsion arising from a pre-concentrate according to Example 1 of the present invention is represented by Figure 2.

Example 8

Verification of Bioavailability of Medicinal Products on Base of Pre-concentrate of Gel Emulsion

The composition according to Example 1 was compared with the commercially available microemulsion product Neoral® oral solution. The composition according to Example 1 was given clinical code L363, Neoral® oral solution was tested under code L352.

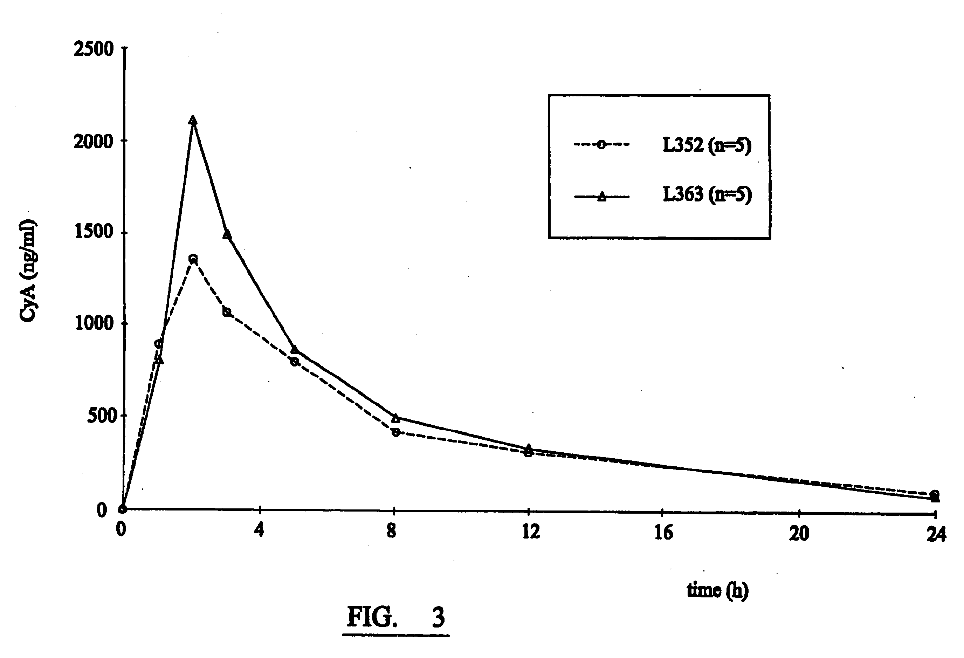

Pharmacokinetics were compared after single-dose administration of 100 mg cyclosporine to five beagle dogs in a two-phase experiment. Males of 12 - 36 months of age and weight 9 - 15 kg were fed using a standard pellet diet in quantity 300 g per day with water ad libitum. The product was administered after 18 hour fasting. Blood samples were collected from the antebrachial vein in intervals of 0, 1, 2, 3, 5, 8, 12 and 24 hour. The blood samples were stabilized using complexone and kept in a refrigerator until analysis was performed by non-specific radioimmunoassay. Comparison of mean bioavailabilities represented by mean values of cyclosporin A blood concentration is shown in Figure 3. It is clear from the comparison that bioavailability of products based on a gel emulsion pre-concentrate which created a dispersion of non-spherical particles of mean size 0.2 - 500 μm after dilution with water, was comparable or higher than that of products forming microemulsion of average size of particles about 100 nm.

Example 9

Fills for Soft Gelatin Capsules Containing Paclitaxel:

The following ingredients were employed.

| a) | paclitaxel | 78.75 mg |
|----|------------|----------|
| b) | polyglyceryl-10-mono-dioleate | 205.00 mg |
| c) | polyglyceryl-3-monooleate | 129.50 mg |
| c) | oleyl alcohol | 205.00 mg |
| d) | macrogol(1760) hydrogenated ricine-oleic glyceride | 302.00 mg |
| e) | ethanol | 129.50 mg |

Example 10

Composition of Soft Gelatin Capsules

The following ingredients were employed.

| a) | paclitaxel | 78.75 mg |
|----|------------|----------|
| a) | [3'ketoMBmt][1]-[Val][2]-cyclosporin | 52.50 mg |
| b) | polyglyceryl-10-mono-dioleate | 187.50 mg |
| c) | oleyl alcohol | 187.50 mg |
| c) | polyglyceryl-3-monooleate | 112.50 mg |
| d) | macrogol(1760) hydrogenated ricine-oleic glyceride | 302.00 mg |
| e) | ethanol | 129.50 mg |

Example 11

Fill for Soft Gelatin Capsules Containing Nifedipine

The following ingredients were employed.

| a) | nifedipine | 20.00 mg |
|----|------------|----------|
| b) | polyglyceryl-10-mono-dioleate | 205.00 mg |
| c) | polyglyceryl-3-monooleate | 129.50 mg |
| c) | oleyl alcohol | 205.00 mg |
| d) | macrogol(1760) hydrogenated ricine-oleic glyceride | 302.00 mg |
| e) | ethanol | 129.50 mg |

Examples 12 - 17

Table 1 gives further examples of preparations illustrating the invention. The method of preparation was identical to that of Example 1.

**Table 1**

| Example No/Component | A | B | $C_1$ | $C_2$ | D | E |
|---|---|---|---|---|---|---|
| 10 | 10.0 | 19.0 | 19.0 | 12.0 | 28.0 | 12.0 |
| 11 | 10.0 | 23.0 | 19.0 | 15.0 | 28.0 | 5.0 |
| 12 | 10.0 | 13.0 | 19.0 | 8.0 | 28.0 | 20.0 |
| 13 | 0.1 | 5.0 | 19.9 | 15.0 | 50.0 | 10.0 |
| 14 | 10.0 | 37.0 | 19.0 | 12.0 | 10.0 | 12.0 |
| 15 | 10.0 | 1.0 | 19.0 | 30.0 | 28.0 | 12.0 |
| 16 | 0.1 | 21.1 | -- | 34.7 | 31.1 | 13.0 |
| 17 | 30.0 | 10.0 | 15.0 | 6.0 | 22.0 | 17/0 |

The following raw materials were used in Examples 10 - 17:

| | |
|---|---|
| A | -cyclosporin A |
| B | -polyglyceryl-10-mono-dioleate (mixture of mono & dioleates) |
| $C_1$ | -oleyl alcohol |
| $C_2$ | -polyglyceryl-3-monoleate |
| D | -macrogol(1760) hydrogenated ricine-oleic glyceride |
| E | -ethanol |

Example 18

Assessment of Bioavailability and Size Distribution of Particles

A bioavailability study on 12 healthy volunteers was compared bioavailability of two different formulations in soft gelatine capsules each containing 100 mg of cyclosporine (Formulation A-GEM101 and Formulation B-GEM304). These gave a dispersion within the range 1-150 $\mu$m with Noreal® 100 mg capsules (Formulation C). Visual observation of the novel drug delivery system and precise evaluation of the particle size distributions were carried out.
Based on the visual observation the novel system was referred to as GEM (Gel based Emulsion).

Composition of Cyclosporin Containing Capsule Fills: Formulation A - GEM 101:

| | | |
|---|---|---|
| a) | cyclosporin A | 1 020 g |
| b) | polyglyceryl-10-monooleate | 2 040 g |
| c) | polyglyceryl-3-monooleate | 3 380 g |
| d) | macrogol (1760) hydrogenated ricine-oleic glyceride | 3 000 g |
| e) | ethanol | 1 330 g |

Formulation B -GEM 304:

| | | |
|---|---|---|
| a) | cyclosporin A | 1 020 g |
| b) | polyglyceryl-10-monooleate | 2 630 g |
| c) | polyglyceryl-3-monooleate | 1 580 g |
| c) | oleyl alcohol | 1 105 g |
| d) | macrogol (1760) hydrogenated ricine-oleic glyceride | 2 450 g |
| e) | ethanol | 1 300 g |

Particle Size Distributions

The particle size distributions of the novel GEM formulations were valuated using a Mastersizer Micro, version 2.18 (Malvern Instruments Ltd). Histograms of particle size distribution of Formulation A (GEM101) and Formulation B (304) showed that the effective diameter of Formulation A (resp. B) deduced from the histogram was 92.05 $\mu$m (36.23 $\mu$m).

Bioequivalence Study Design

An open-label, randomised 3-period crossover study was designed for 12 healthy Caucasian male volunteers, 18 - 45 years of age and with body weights $\pm$ 10% of their ideal weights. The test medications and the reference medication were administered in a randomised sequence as single oral doses in the fasted condition. Each dose contained 200 mg cyclosporin (two capsules of 100 mg). The duration of the washout period between treatments was at least 7 days. In each study period, 14 blood samples were to be taken before administration and 20, 40, 60 min, and 1.5, 2, 2.5, 3, 4, 5, 6, 8, 12 and 24 hours after administration. Adverse events were monitored during the entire study.
Blood was taken from the antecubital vein into EDTA plastic tubes (Sarstedt Monovettes). The samples were deep-frozen (-20 ˚C).
Cyclosporine whole blood concentrations were determined by means of a specific RIA. $AUC_{(0--)}$ and Cmax were defined as the primary variables for the evaluation of bioavailability. $AUC_{(0-t)}$, tmax, t1/2, were secondary variables.
From the concentration/time data of the parent compound, the pharmacokinetic parameters were determined for each individual data set by means of non-compartmental analysis using TopFit 2.0.
Cmax and tmax were to be taken directly from the observed concentration-time data. The elimination rate constant (kel) was calculated by log-linear least squares regression analysis of the terminal part of the plasma concentration-time curve. The area under the concentration-time curve (AUC0-t) was calculated up to the last measurable concentration-time point (t) by the linear trapezoidal rule. Extrapolation to infinity (AUC0-t, AUC0-∞) was done by dividing the last observed concentration by elimination rate constant.
Summary of pharmacokinetic data:

| Parameter | T1/2 [h] | Tmax [h] | Cmax [ng/ml] | AUC(0-t) [ng*h/ml] | AUC(0-inf) [ng*h/ml] |
|---|---|---|---|---|---|
| **Formulation: A** | | | | | |
| Arit.Mean | 6.24 | 1.33 | 1372.69 | 4631.75 | 4861.85 |
| S.D. | 1.3 | 0.33 | 351.28 | 1204.56 | 1241.87 |
| Geom.Mean | 6.12 | 1.3 | 1329.84 | 4483.35 | 4712.35 |
| Minimum | 4.06 | 1 | 908.1 | 2635.32 | 2873.57 |
| Maximum | 8.24 | 2 | 1930.3 | 6432.76 | 6684.33 |
| **Formulation: B** | | | | | |
| Arit.Mean | 6.41 | 1.5 | 1196.49 | 4430.33 | 4696.56 |
| S.D. | 1.3 | 0.48 | 308.26 | 1032.91 | 1143.13 |
| Geom.Mean | 6.29 | 1.43 | 1161.84 | 4326.15 | 4576.94 |
| Minimum | 4.21 | 1 | 851.8 | 3130.66 | 3254.08 |
| Maximum | 8.93 | 2.5 | 1785 | 6206.13 | 6643.15 |
| **Formulation: C / Reference** | | | | | |
| Arit.Mean | 6.13 | 1.33 | 1358.95 | 4647.01 | 4887.55 |
| S.D. | 1.32 | 0.33 | 380.35 | 1358.41 | 1430.5 |
| Geom.Mean | 5.99 | 1.3 | 1307.19 | 447208 | 4705.55 |
| Minimum | 3.92 | 1 | 820.7 | 2953.47 | 3028.58 |
| Maximum | 7.87 | 2 | 1805.3 | 7330.08 | 7686.89 |

Example 19

Visualisation of Different Formulations

Different shapes of particles can be obtained by dispersal of formulations disclosed in this application. The following compositions when diluted gave dispersions of polymorphic gel particles. The visualisation technique was as described in Example 5.

Formulations A and B from Example 18 were visualized. A discrepancy between the measured (Mastersizer Micro: example 18) and observed particle sizes was caused by use of different dispersal techniques and by averaging of the measured values. Whilst the sample measured by Mastersizer Micro is continuously mixed by high speed mixer, a sample observed by an optical microscope was softly shaken by hand before putting under optical microscope.

The following formulations were also observed and visualised:

Formulation C

| a) | cyclosporin A | 9.5% |
|---|---|---|
| b) | polyglyceryl-10-monooleate | 40.0% |
| c) | polyglycerol-3-isostearate | 10.0% |
| d) | macrogol (1760) hydrogenated ricine-oleic glyceride | 28.0% |
| e) | ethanol | 12.5% |

Formulation D

| a) | cyclosporin A | 10.0 % |
|---|---|---|
| b) | polyglyceryl-10-monolaurate | 10.0% |
| c) | polyglycerol-3-oleate | 40.0% |
| d) | macrogol (1760) hydrogenated ricine-oleic glyceride | 28.0% |
| e) | ethanol | 12.0% |

Formulation E

| a) | cyclosporin A | 10.0 % |
|---|---|---|
| b) | polyglyceryl-10-monolaurate | 27.0 % |
| c) | polyglycerol-3-heptaoleate | 31.0 % |
| d) | macrogol (1760) hydrogenated ricine-oleic glyceride | 20.0 % |
| e) | ethanol | 12.0 % |

Example 20:

Assessment of viscosity of arising gel phases.

Compositions disclosed in this specification may exhibit an increase in viscosity in contact with water or aqueous solutions. This feature is particularly important for ensuring bioavailability of an active substance incorporated in such formulation. The viscosities of compositions from Examples 18 and 19 evaluated experimentally.

The rheological properties of chosen compositions were studied on a rotary viscometer Brookfield DV-III under constant conditions (temperature = 30˚C, spindle SC 4 - 27, ultrathermostat Brookfield TC 500, Rheocalc program, 1.3 version).

A standard dilution was used to compare the ability to form a gel phase. Each sample was diluted 1 : 1 (by volume) with water. The viscosity of the diluted sample was evaluated using an up/down symmetric rheological program. All diluted samples were found to be non-Newtonian liquids. Undiluted samples had characteristics of standard (Newtonian) liquids. The samples were compared at the same Shear Rate. Findings are summarised in the table below:

**Rheological parameters at the constant Shear Rate = 1.70 sec$^{-1}$:**

| Formulation (dilution status) | Shear Stress ($N/m^2$) | Viscosity (mPa.s) |
|---|---|---|
| Formulation A (undiluted) | 0.34 | 200 |
| Formulation A (diluted) | 3.91 | 2300 |
| Formulation C (diluted) | 6.97 | 4100 |
| Formulation D (diluted) | 17.2 | 10100 |
| Formulation E (diluted) | 1.53 | 900 |

It was conclude that viscosity of the novel systems could be increased by at least 5x when contacted with water or aqueous solution. Such viscosity increases may have positive impact on the adhesion of the nascent phase and consequently provide an improved bioavailability.

**Claims**

1.   A pharmaceutical formulation for oral or topical administration including

a) 0.1 to 30.0% of one or more hydrophobic active ingredients;
b) 0.1 to 60.0% of one or more gelators selected from polyglycerol esters of fatty acids of formula (1)

$$CH_2OR-CHOR-CH_2O-[CH_2CHOR-CH_2O-]_NCH_2-CHOR-CH_2OR \qquad (1)$$

wherein n is an integer from 4 to 13 and R is H or CO.R' wherein R' is $C_{8-22}$ saturated, unsaturated or hydroxylated alkyl and wherein at least one group R is not hydrogen; having a HLB value not less than 10.
c) 0.1 to 60.0% of one or more gel-creating substances selected from polyglycerol esters of fatty acids and/or unsaturated fatty acids of formula (2)

$$CH_2OR-CHOR-CH_2O-[CH_2CHOR-CH_2O]_NCH_2-CHOR-CH_2OR \qquad (2)$$

wherein n is an integer from 0-10 and R = H or CO.R" wherein R" is $C_{8-22}$ saturated, unsaturated or hydroxylated alkyl, and wherein while at least one group R is not hydrogen; having a HLB value not greater than 9;
d) 1.0 to 60% of one or more co-gelator substances selected from triglyceride macrogol glycerol esters, partial glycerides or fatty acids or macrogol esters of fatty acids in which the average quantity of reacted ethylene oxide in the synthesis of these substances ranges between 50 to 150 mols and concurrently the ratio between components b) and d) is from 0.1:1 to 10:1.
e) 5.0 to 30% of one or more $C_2$ to $C_4$ alcohols;

wherein the above percentages are selected to total 100%;
and wherein upon dilution with water the formulation forms a dispersion of polymorphous gel particles having a dimension of 0.2 to 500$\mu$m.

2.   A pharmaceutical formulation as claimed in claim 1, wherein the ratio of a:c and/or a:e is in the range 0.001:1 to 10:1.

3.   A formulation as claimed in claim 1 or 2 wherein R' is $C_{16-18}$ saturated or unsaturated alkyl.

4.   A formulation as claimed in claim 3, wherein R is selected from the group consisting of oleates, linoleate stearate, linolate, myristate, laurate and mixtures thereof.

5.   A formulation as claimed in claim 4, where component b) is selected from: polyglyceryl-10-esters of fatty acids.

6.   A formulation as claimed in claim 5, wherein R" is $C_{16-18}$ saturated or unsaturated alkyl.

7.   A formulation as claimed in claim 6, wherein R is selected from the group of oleate, linoleate, stearate, isostearate, linolate, myristate, laurate and mixtures thereof.

8.   A formulation as claimed in any preceding claim, wherein component c) is selected from: polyglyceryl-3-esters of oleic acid.

9. A formulation as claimed in any preceding claim, wherein component d) is macrogol glycol hydrogenated castor oil.

10. A formulation as claimed in any preceding claim, wherein component b) is selected from: polyglyceryl-10-esters of oleic acid; component c) is selected from polyglyceryl-3-esters of oleic acid; and component d) is macrogol (1760) glycerol hydrogenated caster oil.

11. A formulation as claimed in any preceding claim, wherein the component a) is selected from cyclosporins especially cyclosporin A, cyclosporin D or cyclosporin G, wherein the ratio of components a:c+e is 1.001:1 to 1.5:1.

12. A formulation as claimed in any preceding claim wherein the alcohol is ethanol.

13. A formulation as claimed in any preceding claim, further including excipients to modify the physical, chemical, microbial stability, organoleptic or physical processing properties of the formulation.

14. A pharmaceutical dosage form comprising a gelatin capsule containing a formulation as claimed in any preceding claim.

**Patentansprüche**

1. Pharmazeutische Formulierung für die orale oder topische Verabreichung, enthaltend

a) 0,1 bis 30,0% eines oder mehrerer hydrophober aktiver Inhaltsstoffe,
b) 0,1 bis 60,0% eines oder mehrerer Gelbildner, ausgewählt unter Polyglycerolestern von Fettsäuren der Formel (1)

$$CH_2OR\text{-}CHOR\text{-}CH_2O\text{-}[CH_2CHOR\text{-}CH_2O\text{-}]_nCH_2\text{-}CHOR\text{-}CH_2OR \qquad (1)$$

worin n eine ganze Zahl von 4 bis 13 ist und R H oder CO.R' ist, wobei R' gesättigtes, ungesättigtes oder hydroxyliertes $C_{8\text{-}22}$-Alkyl ist und wobei wenigstens eine Gruppe R nicht Wasserstoff ist, mit einem HLB-Wert von nicht kleiner als 10,
c) 0,1 bis 60,0% einer oder mehrerer gelerzeugender Substanzen, ausgewählt unter Polyglycerolestern von Fettsäuren und/oder ungesättigten Fettsäuren der Formel (2)

$$CH_2OR\text{-}CHOR\text{-}CH_2O\text{-}[CH_2CHOR\text{-}CH_2O]_nCH_2\text{-}CHOR\text{-}CH_2OR \qquad (2)$$

worin n eine ganze Zahl von 0-10 ist und R = H oder CO.R" ist, wobei R" gesättigtes, ungesättigtes oder hydroxyliertes $C_{8\text{-}22}$-Alkyl ist und wobei wenigstens eine Gruppe R nicht Wasserstoff ist, mit einem HLB-Wert von nicht größer als 9,
d) 1,0 bis 60% einer oder mehrerer Co-Gelbildnersubstanzen, ausgewählt unter Triglyceridmacrogolglycerolestern, unvollständig veresterten Glyceriden oder Fettsäuren oder Macrogolestern von Fettsäuren, bei denen die mittlere Menge an umgesetztem Ethylenoxid in der Synthese dieser Substanzen im Bereich zwischen 50 bis 150 Mol liegt und gleichzeitig das Verhältnis zwischen den Komponenten b) und c) 0,1 : 1 bis 10 : 1 beträgt,
e) 5,0 bis 30% eines oder mehrerer $C_2$- bis $C_4$-Alkohole,

wobei die obigen Prozentanteile so ausgewählt sind, daß sie zusammen 100% ergeben,
und wobei bei Verdünnung mit Wasser die Formulierung eine Dispersion von polymorphen Gelpartikeln mit einer Dimension von 0,2 bis 500 $\mu$m bildet.

2. Pharmazeutische Formulierung nach Anspruch 1, wobei das Verhältnis von a : c und/oder a : e im Bereich von 0,001 : 1 bis 10 : 1 liegt.

3. Formulierung nach Anspruch 1 oder 2, wobei R' gesättigtes oder ungesättigtes $C_{16\text{-}18}$-Alkyl ist.

4. Formulierung nach Anspruch 3, wobei R aus der Gruppe ausgewählt ist, bestehend aus Oleaten, Linoleat, Stearat, Linolat, Myristat, Laureat und Gemischen davon.

**5.** Formulierung nach Anspruch 4, wobei Komponente b) unter Polyglyceryl-10-estern von Fettsäuren ausgewählt ist.

**6.** Formulierung nach Anspruch 1 oder Anspruch 2, wobei R" gesättigtes oder ungesättigtes $C_{16-18}$-Alkyl ist.

**7.** Formulierung nach Anspruch 6, wobei R aus der Gruppe von Oleat, Linoleat, Stearat, Isostearat, Linolat, Myristat, Laureat und Gemischen davon ausgewählt ist.

**8.** Formulierung nach einem der vorangegangenen Ansprüche, wobei Komponente c) unter Polyglyceryl-3-estern von Ölsäure ausgewählt ist.

**9.** Formulierung nach einem der vorangegangenen Ansprüche, wobei Komponente d) Macrogolglycol-hydriertes Castoröl ist.

**10.** Formulierung nach einem der vorangegangenen Ansprüche, wobei Komponente b) unter Polyglyceryl-10-estern von Ölsäure ausgewählt ist, Komponente c) unter Polyglyceryl-3-estern von Ölsäure ausgewählt ist und Komponente d) Macrogol (1760) Glycerol-hydriertes Castoröl ist.

**11.** Formulierung nach einem der vorangegangenen Ansprüche, wobei Komponente a) unter Cyclosporinen, insbesondere Cyclosporin A, Cyclosporin D oder Cyclosporin G, ausgewählt ist,
wobei das Verhältnis der Komponenten a : c + e 1,001 : 1 bis 1,5 : 1 beträgt.

**12.** Formulierung nach einem der vorangegangenen Ansprüche, wobei der Alkohol Ethanol ist.

**13.** Formulierung nach einem der vorangegangenen Ansprüche, welche weiterhin Hilfsstoffe zum Modifizieren der physikalischen, chemischen oder mikrobiellen Stabilität oder der organoleptischen oder physikalischen Verarbeitungseigenschaften der Formulierung umfaßt.

**14.** Pharmazeutische Dosierungsform, welche eine Gelatinekapsel umfaßt, die eine Formulierung nach einem der vorangegangenen Ansprüche enthält.


**Revendications**

**1.** Formulation pharmaceutique pour une administration par voie orale ou topique comprenant:

a) 0,1 à 30,0% d'un ou plusieurs principes actifs hydrophobes;
b) 0,1 à 60,0% d'un ou plusieurs gélifiants choisis parmi les esters de polyglycérol et d'acides gras de formule (1):

$$CH_2OR\text{-}CHOR\text{-}CH_2O\text{-}[CH_2CHOR\text{-}CH_2O]_nCH_2\text{-}CHOR\text{-}CH_2OR \qquad (1)$$

dans laquelle

n représente un nombre entier compris entre 4 et 13 et
R représente un atome d'hydrogène ou COR', R' représentant un groupe alkyle en $C_8$ à $C_{22}$ saturé, insaturé ou hydroxylé

et dans laquelle au moins un des groupes R ne représente pas un atome d'hydrogène; lesdits gélifiants présentant une valeur HLB non inférieure à 10;
c) 0,1 à 60,0% d'une ou plusieurs substances, formant un gel, choisies parmi les esters de polyglycérol et d'acides gras et/ou d'acides gras insaturés de formule (2):

$$CH_2OR\text{-}CHOR\text{-}CH_2O\text{-}[CH_2CHOR\text{-}CH_2O]_nCH_2\text{-}CHOR\text{-}CH_2OR \qquad (2)$$

dans laquelle

n représente un nombre entier compris entre 0 et 10 et
R = H ou COR", R" représentant un groupe alkyle en $C_8$ à $C_{22}$ saturé, insaturé ou hydroxylé,

et dans laquelle au moins un des groupes R ne représente pas un atome d'hydrogène; lesquelles substances présentent une valeur HLB ne dépassant pas 9;

d) 1,0% à 60% d'une ou plusieurs substances co-gélifiantes choisies parmi les esters de PEG-triglycéride, les glycérides partiels ou les esters d'acides gras de PEG d'acides gras dans lesquelles la quantité moyenne d'oxyde d'éthylène ayant réagi lors de la synthèse de ces substances est comprise entre 50 et 150 moles et simultanément le rapport entre les composants b) et d) est compris entre 0,1/1 et 10/1;

e) 5,0% à 30% d'un ou plusieurs alcools en $C_2$ à $C_4$;

formulation dans laquelle les pourcentages ci-dessus sont exprimés par rapport à un total de 100%; et laquelle formulation, après dilution dans l'eau, formant une dispersion de particules de gel polymorphe ayant une dimension de 0,2 à 500 $\mu$m.

2. Formulation pharmaceutique selon la revendication 1, dans laquelle le rapport a/c et/ou a/e est dans une gamme comprise entre 0,001/1 et 10/1.

3. Formulation selon la revendication 1 ou 2, dans laquelle R' représente un groupe alkyle en $C_{16}$ à $C_{18}$, saturé ou insaturé.

4. Formulation selon la revendication 3, dans laquelle R est choisi dans le groupe constitué par les oléates, stéarates, linoléates, linolates, myristates, laurates et leurs mélanges

5. Formulation selon la revendication 4, dans laquelle le composant b) est choisi parmi les polyglycényl-10-esters d'acides gras.

6. Formulation selon la revendication 5, dans laquelle R" représente un groupe alkyle en $C_{16}$ à $C_{18}$, saturé ou insaturé.

7. Formulation selon la revendication 6, dans laquelle R est choisi dans le groupe constitué par les oléates, linoléates, stéarates, isostéarates, linolates, myristates, laurates et leurs mélanges.

8. Formulation selon l'une quelconque des revendications précédentes, dans laquelle le composant c) est choisi parmi: les polyglycéryl-3-esters de l'acide oléique.

9. Formulation selon l'une quelconque des revendications précédentes, dans laquelle le composant d) est l'huile de ricin hydrogénée de PEG.

10. Formulation selon l'une quelconque des revendications précédentes, dans laquelle le composant b) est choisi parmi les polyglycéryl-10-esters de l'acide oléique; le composant c) est choisi parmi les polyglycéryl-3-esters de l'acide oléique; et le composant d) est l'huile de ricin hydrogénée de PEG (1760) glycérol.

11. Formulation selon l'une quelconque des revendications précédentes, dans laquelle le composant a) est choisi parmi les cyclosporines, en particulier la cyclosporine A, la cyclosporine D ou la cyclosporine G, et dans laquelle le rapport des composants a/(c+e) est compris entre 1,001/1 et 1,5/1.

12. Formulation selon l'une quelconque des revendications précédentes, dans laquelle l'alcool est l'éthanol.

13. Formulation selon l'une quelconque des revendications précédentes, comprenant en outre des excipients pour modifier les propriétés de traitement physique ou organoleptique, de stabilité microbienne, physiques, chimiques de la formulation.

14. Forme de dosage pharmaceutique comprenant une gélule contenant une formulation selon l'une quelconque des revendications précédentes.

FIG.    1

FIG.    2

FIG.    3

Formation A

## FIG. 4

Formation B

## FIG. 5

Formation C

## FIG. 6

Formation D

## FIG. 7

Formation E

# FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 2015339 A
- GB 2222770 A
- GB 2270842 A
- GB 2278780 A
- GB 2228198 A
- DE 4322826 A
- GB 2248615 A

- WO 9805309 A
- WO 9726003 A
- EP 670715 A
- EP 334777 A
- CZ 283516 A
- EP 314689 A
- EP 126751 A

**Non-patent literature cited in the description**

- **G L AMIDON.** *Biopharmaceutics Drug Classification and International Drug Regulation,* 1996, 15-30

- *Pharmaceopeial Forum,* 1998, vol. 24 (3), 6155